# EUROPEAN PATENT APPLICATION

(11) **EP 1 125 552 A1**
(43) Date of publication of application: **22.08.2001**
(21) Application number: 00650010.2
(22) Date of filing: 18.02.2000
(51) Int. Cl.: A61B 17/02, A61B 17/34

(54) **Surgical retractor**

(71) Applicant: ATROPOS LIMITED, Dublin 2 (IE)
(72) Inventor: Butler, John, Blackrock, County Dublin (IE); Young, Derek William, Blackrock, County Dublin (IE); Gill, Aoibheann, Lanesboro, County Longford (IE); Reid, Alan, Clontarf, Dublin 3 (IE); Bonadio, Frank, Bray, County Wicklow (IE)
(74) Representative: O'Brien, John Augustine

(57) **Abstract**

A retractor 1 for retracting the margins 2 of a wound opening 3 comprises an inner O-ring 5 attached to a sleeve 6 at a distal end and a reinforcing ring 9 mounted to the proximal end of the sleeve 6. The sleeve 6 is led between an inner annular ring 10 and a corresponding recess 16 in an outer ring 11. The outer ring 11 has anchor elements 20 over which the proximal end of the sleeve 6 is led to anchor the sleeve. To retract a wound opening 3 the sleeve 6 is pulled upwardly while the guide rings 10, 11 are moved downwardly. This pulls the inner O-ring 5 against the inside of the tissue adjacent the wound opening 3, pushes the rings 10, 11 down against the outside tissue and retracts the wound opening 3. The sleeve 6 is manipulated locally for maximum retraction efficiency.

## Description

### Introduction

The invention relates to a retractor for retracting the margins of an incision or a natural bodily orifice to provide maximum exposure of the organ or body structures for examination and/or access for surgical procedures.

### Statements of the Invention

According to the invention there is provided a retractor comprising:-
an inner mounting means for mounting through a wound or body opening;
connecting means having a distal end and a proximal end;
the connecting means being mounted to the inner mounting means;
external guide means for the connecting means;
the connecting means being movable relative to the guide means to retract the opening; and
anchor means for anchoring the connecting means to maintain retraction of the opening.

In a preferred embodiment of the invention the connecting member is a sleeve.

Preferably the guide means comprises an annular ring means.

The annular ring means preferably comprises inner and outer ring parts between which the connecting means is led. In a preferred arrangement the outer ring part includes the anchor means for anchoring the connecting means.

In one embodiment the anchor means comprises anchor formations on the outer ring to which the connecting means is attached on retraction of the opening.

In a preferred embodiment the inner ring means defines a projection for engagement in a complementary recess of the outer ring with the connecting means located therebetween.

Preferably at least portion of one of the ring parts is movable from a rest position in which the connecting member is substantially clamped between the ring parts to a release position in which the connecting member is movable relative to the ring parts.

In one embodiment the outer ring part comprises a plurality of segments which are independently movable to facilitate localised release of the connecting member for adjusting of the retraction force applied at the opening.

Preferably the ring part or segment thereof are manually manipulable between the clamped rest and release positions.

In a preferred embodiment the retractor includes a platform for attachment of another device to the retractor.

Ideally one of the ring parts defines a platform for attachment of another device to the retractor.

Preferably the connecting means includes a proximal reinforcing means. The proximal reinforcing means may be a proximal ring.

Ideally the distal mounting means is an O-ring.

### Brief Description of the Drawings

The invention will be more clearly understood from the following description thereof given by way of example only in which: -
Fig. 1 is an exploded view of a retractor according to the invention;
Fig. 2 is a perspective view of the retractor assembled;
Fig. 3 is a cross sectional view of the retractor in an initial position;
Fig. 4 is a top plan view of the retractor in the position of Fig. 3;
Fig. 5 is a cross sectional view of the retractor in a intermediate position;
Fig. 6 is a cross sectional view of the retractor in a retracting configuration;
Fig. 7 is a top plan view of a the retractor in the configuration of Fig. 5;
Figs. 8 to 10 are cross sectional views illustrating manipulation of the retractor in use;
Fig. 11 is an exploded view of another retractor according to the invention;
Fig. 12 is a perspective view of the retractor of Fig. 11, assembled.
Fig. 13 is a cut-away view of the retractor of Fig. 12;
Fig. 14 is another cut-away view of the retractor of Fig. 13 in another position;
Fig. 15 is a diagrammatic plan view illustrating the manipulation of part of the retractor of Figs. 12 to 14;
Fig. 16 is a cross sectional view of the retractor part in the configuration of Fig. 15;
Fig. 17 is a diagrammatic plan view of the retractor part of Fig. 15 in a release position;
Fig. 18 is a cross sectional view of the retractor part in the configuration of Fig. 17;
Fig. 19 is a perspective view of a hand access device for use with the retractor;
Figs. 20 and 21 are cross sectional views of the retractor with the hand access device of Fig. 19 in position;

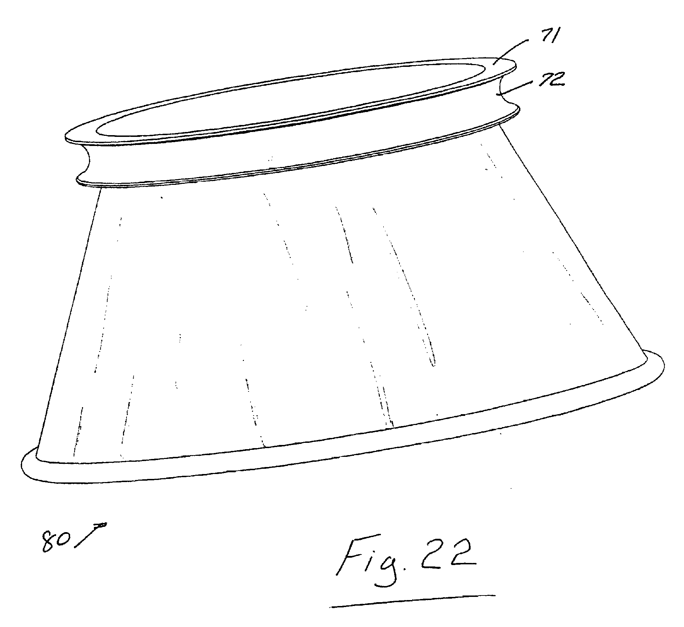

Fig. 22 is a perspective view of a drape for use with the retractor;

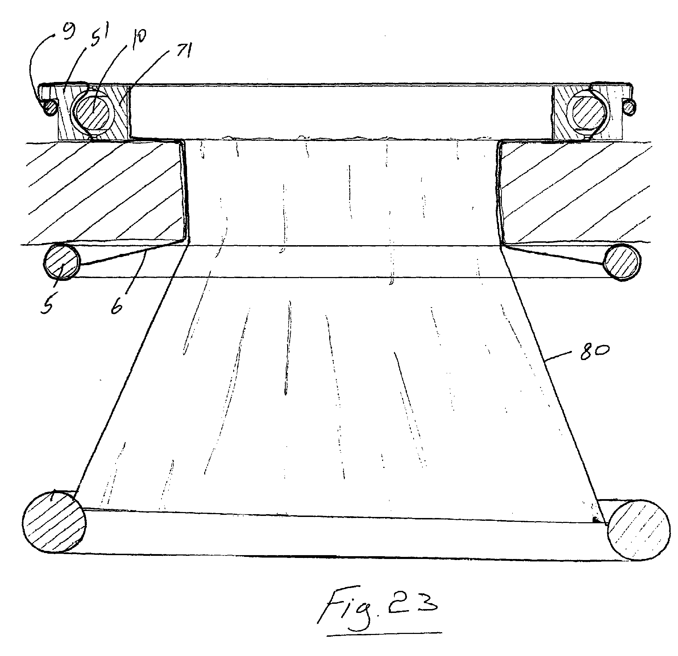

Fig. 23 is a cross sectional view of the retractor with the drape of Fig. 22 in place;

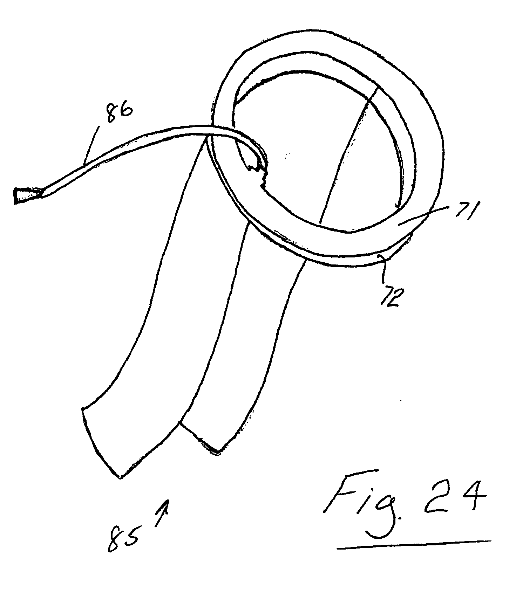

Fig. 24 is a perspective view of a form retaining device for use with the retractor; and

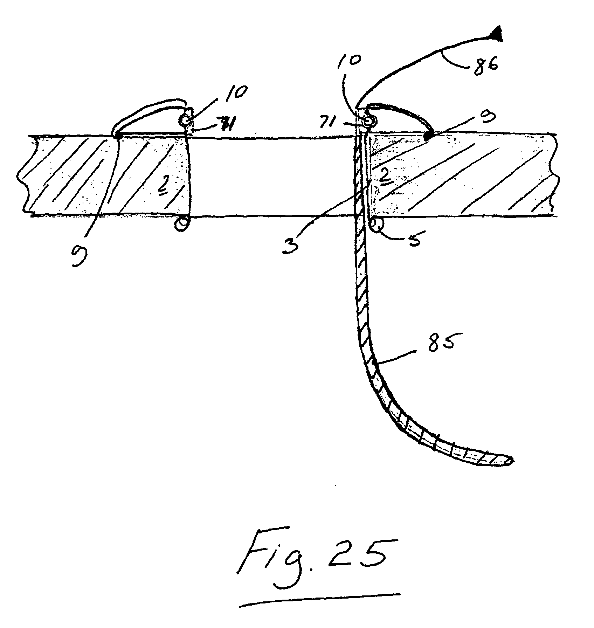

Fig. 25 is a cross sectional view of the retractor with the form retaining device of Fig. 24 in position.

### Detailed Description

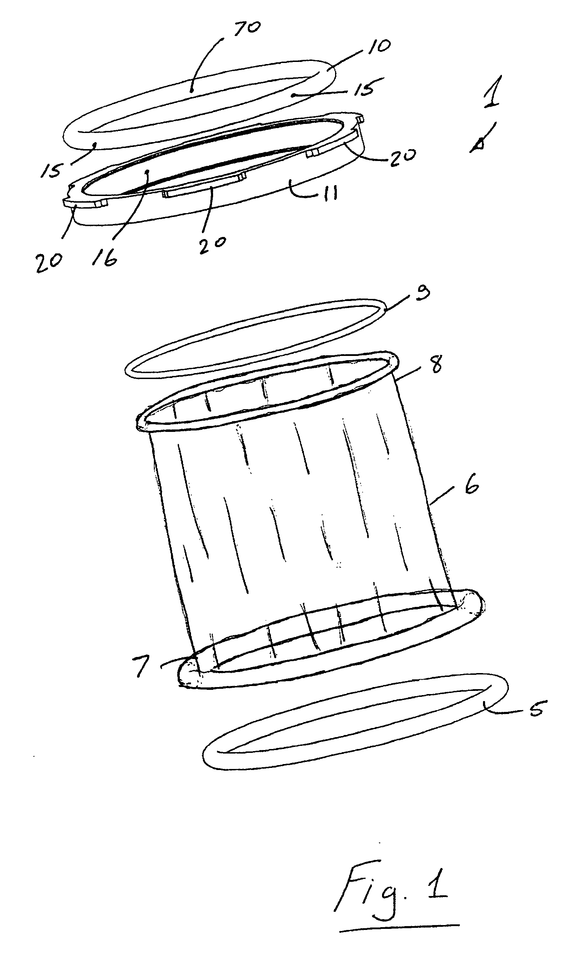

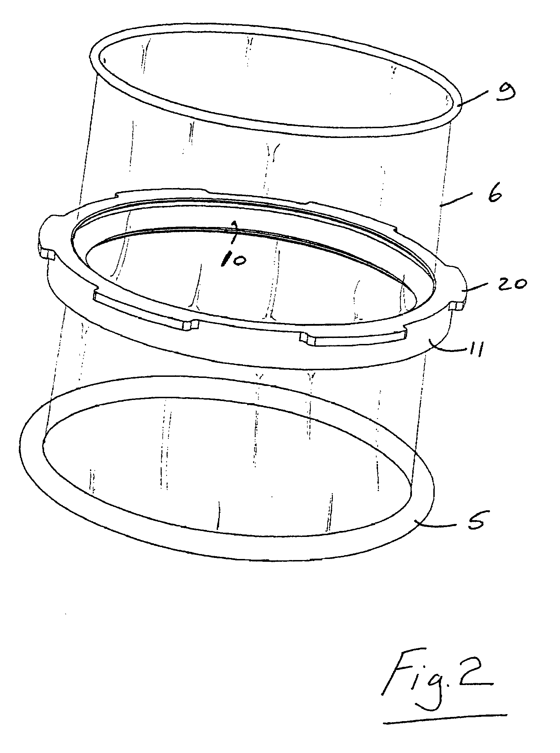

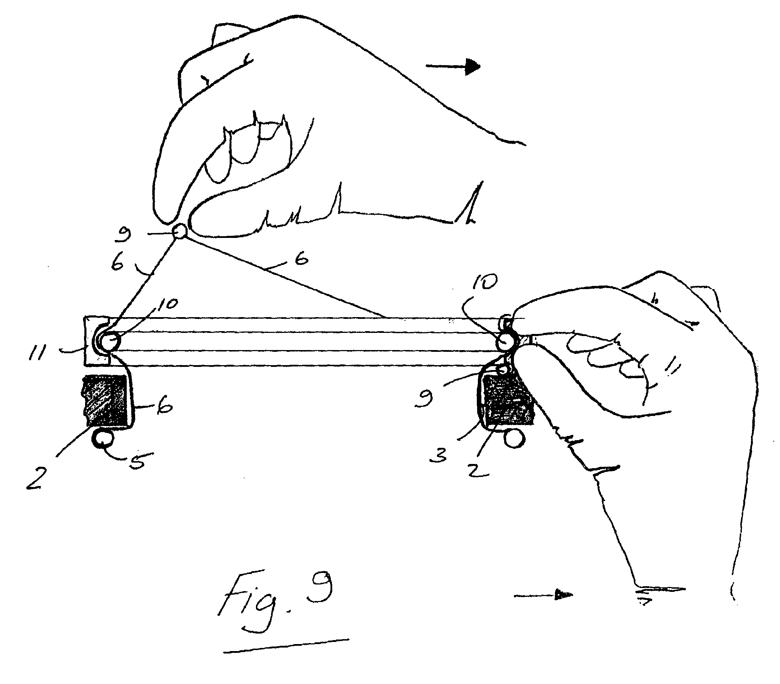

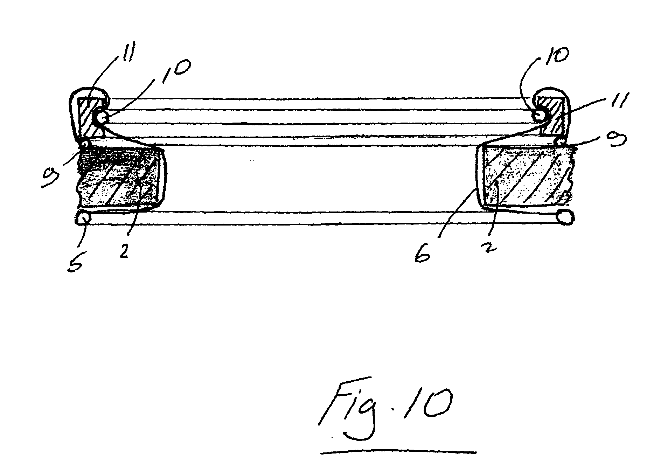

Referring to the drawings and initially to Figs. 1 to 10 thereof there is illustrated a retractor 1 for retracting the margins 2 of a wound opening 3 or a body orifice.

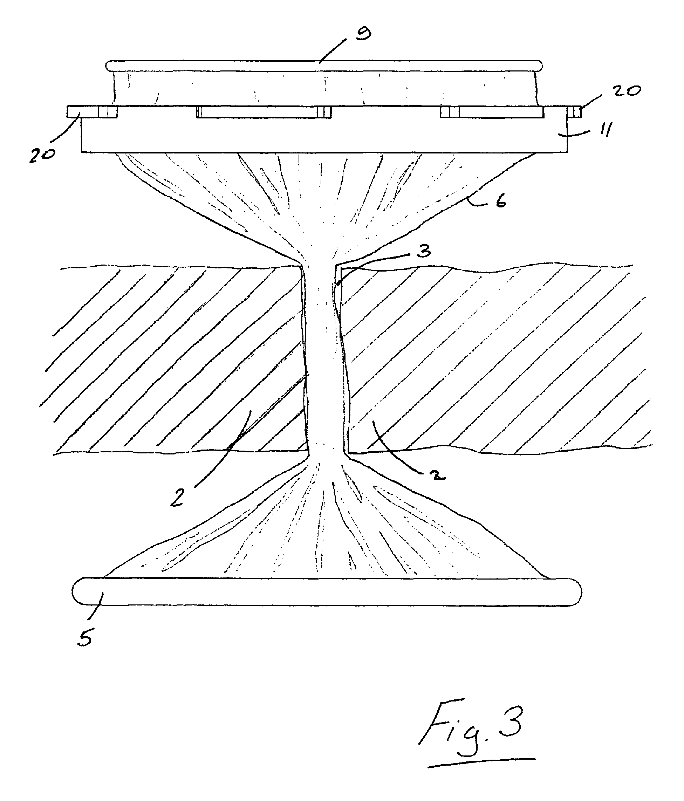

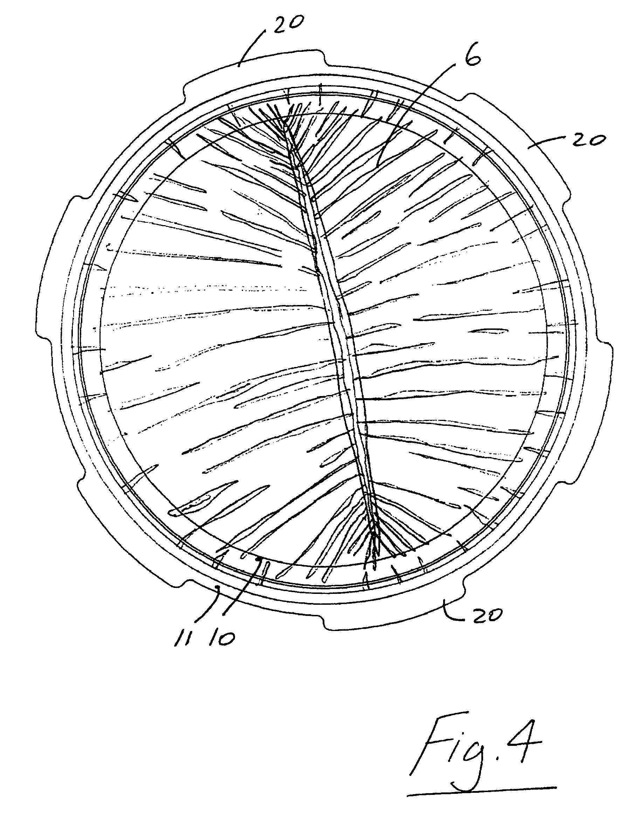

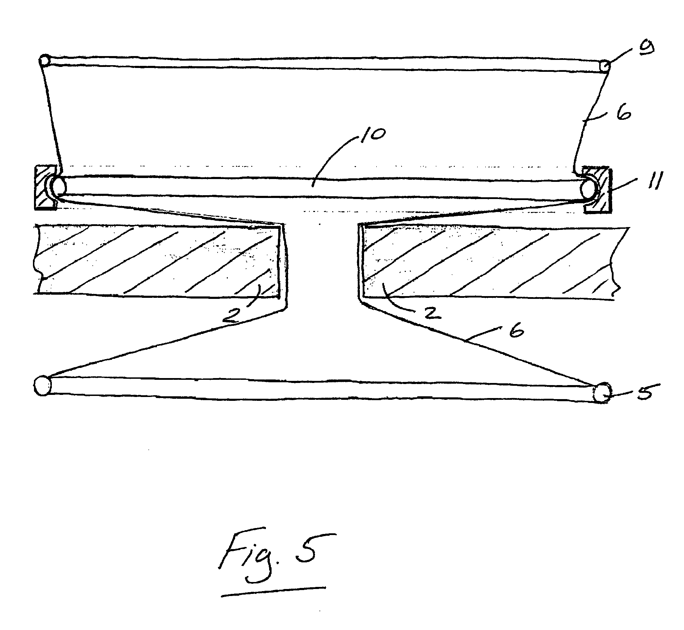

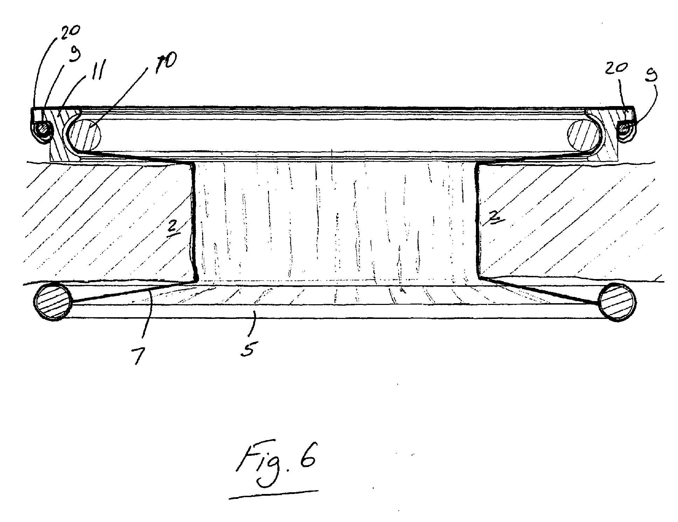

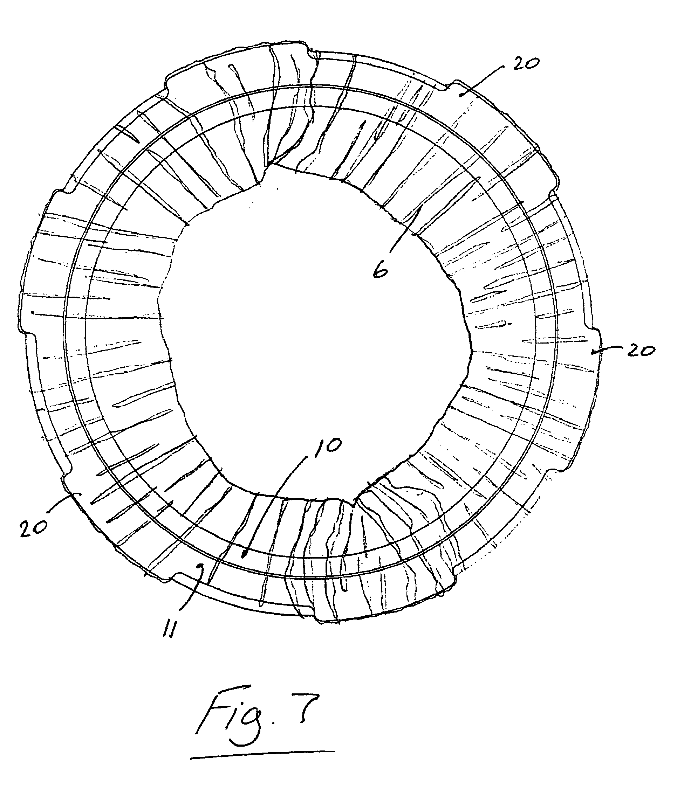

The retractor 1 comprises an inner mounting means, in this case in the form of an O-ring 5 which, in use is squeezed for insertion through an unretracted wound opening. Connecting means in the form of a sleeve 6 has a distal end 7 mounted to the inner O-ring 5 and a proximal end 8 which is reinforced by a flexible reinforcing polymeric ring 9 attached to the sleeve 6. External guide means for guiding movement of the sleeve 6 is provided by an annular ring means which in this case has an inner ring part 10 and an outer ring part 11 between which the sleeve 6 is retained. The inner ring part 10 is of a relatively stiff material and defines an outwardly extending projection 15 which engages a complementary recess 16 in the outer ring part 11 to retain the sleeve 6 therebetween. In this case the ring parts 10, 11 are a relatively loose fit to facilitate controlled application of a retraction force by pulling the sleeve 6 upwardly while moving the ring parts 10, 11 downwardly from the initial insertion configuration illustrated in Fig. 3 and 4 with the incision almost closed, through an intermediate position illustrated in Fig. 5 in which the sleeve 6 has been pulled partially up and the ring parts moved down, to a final retracting position illustrated in Figs. 6 and 7 in which the pulling of the sleeve 6 upwardly has resulted in a controlled retraction force being applied to the margin of the incision.

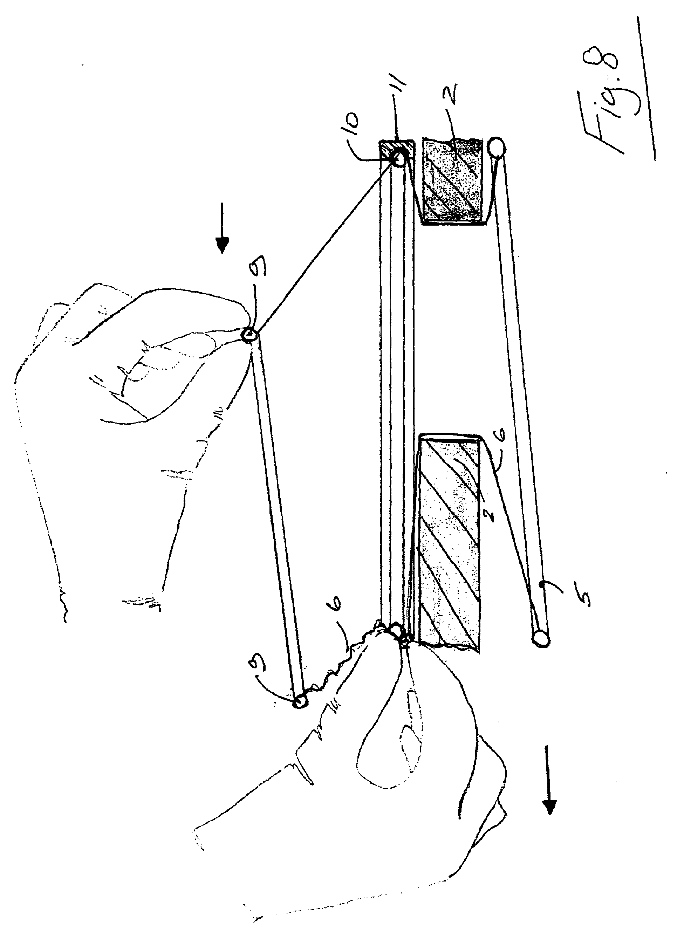

Anchor means for anchoring the sleeve 6 to maintain the retraction force is in this case provided by radially projecting anchor elements 20 on the outer ring 11 over which the pliable sleeve 6 is pulled and retained by the proximal reinforcing ring 9. The separate anchor element 20 and the configuration of the device generally allows the sleeve 6 to be readily manipulated locally as illustrated in Fig. 8 to provide an optimised retraction force. Thus, the manipulation is not simply limited to a single vertical axis but can be carried out in several different directions by manipulation of the sleeve 6, gripping of the ring 19, and localised anchoring of the sleeve 6 to the appropriate anchor element 20. A single retractor may therefore to be used for a wide range of incision sizes and to achieve a range of different localised retraction forces which are required to accommodate the incision, the patient anatomy and the surgical procedure to be performed.

The elastomeric sleeve 6 acts both as a means of wound retraction and wound protection. Polymeric ring 5, 9 are attached to each end of the elastomeric sleeve 6 to act as a means of maintaining the shape of the sleeve 6 at its extremities. The elastomeric sleeve 5 is slidably disposed within another section of the device that consists of two annular components 10, 11 of a material with a low coefficient of friction such as Polytetrafluoroethylene (PTFE). PTFE is a tough, non-resilient material of moderate tensile strength with excellent lubricity. These PTFE components consist of a ring 10 or other annular shape around which slides the second component 11. The second component 11 is of the same annular shape as ring 10 but has a larger diameter and an interior compartmental recess 16 designed so that the ring 10 loosely within the recess 11 and both rings 10, 11 can easily slide relative to each other. The components 10, 11 fit loosely together so that the elastomeric sleeve can fit in the gap therebetween and slide therein.

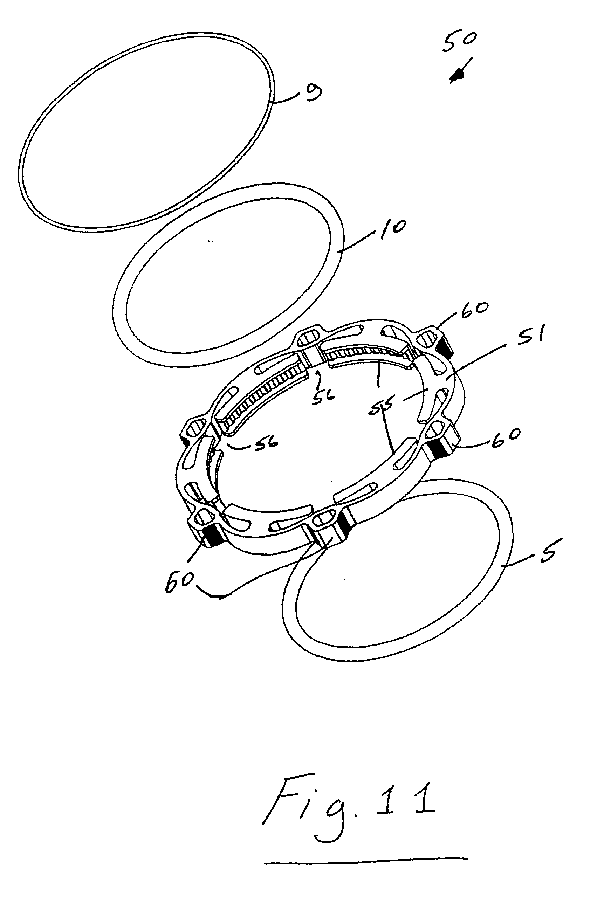

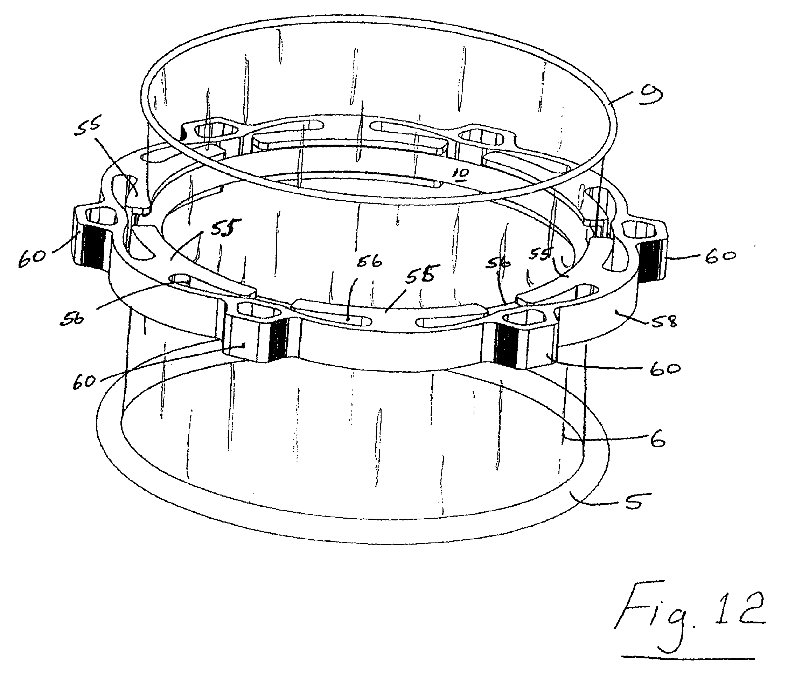

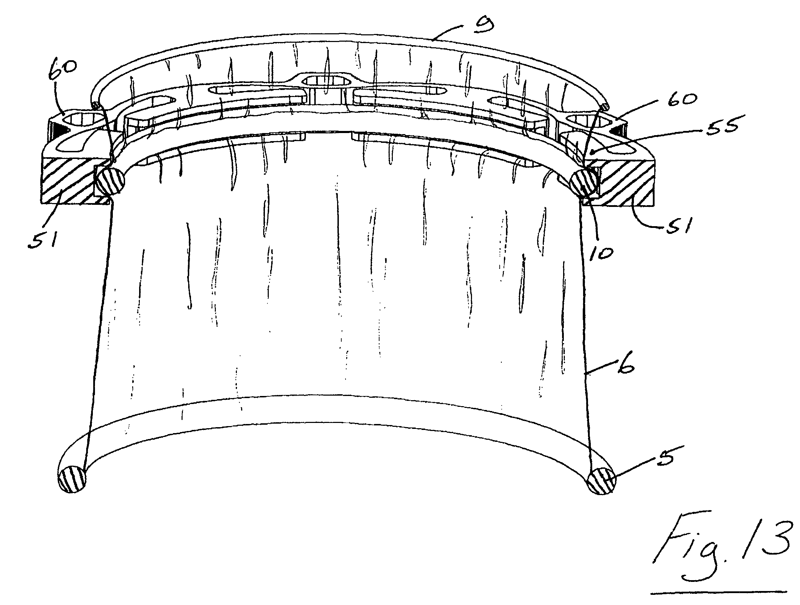

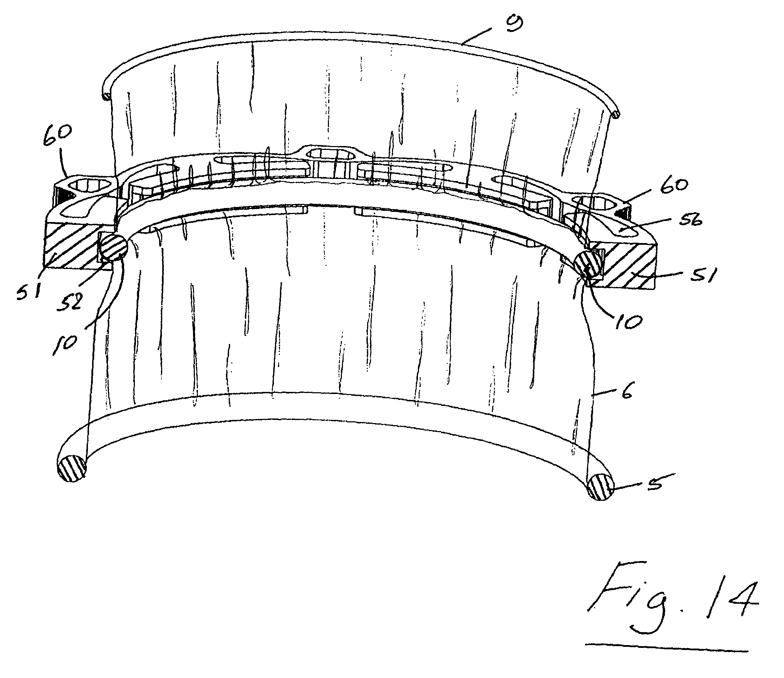

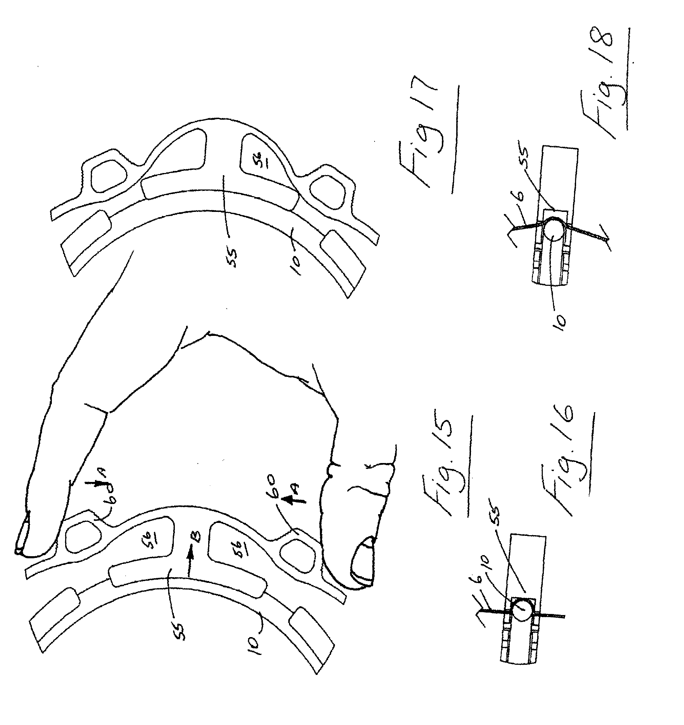

Referring to Figs. 11 to 18 there is illustrated another retractor 50 according to the invention which is similar to the retractor described with reference to Figs. 1 to 10 and like parts are assigned the same reference numerals. In this case an outer ring part 51 is configured so that at least portion of the ring 51 is movable from a rest position (Figs. 15 and 16) in which the sleeve 6 is substantially clamped between the ring parts 10, 51 to a release position (Figs. 17 and 18) in which the sleeve 6 is movable relative to the ring parts 10, 51. The outer ring part 51 has a plurality of circumferentially spaced-apart segments 55 which are independently movable to facilitate localised release of the sleeve 6 for adjustment of the retraction force. Cut-out T-slots 56 are provided between the segments 57 and a main body 58 of the ring 51. Handles 60 project radially outwardly of the main body 58 of the ring intermediate the segment 55. In use, adjacent handles 60 are gripped as illustrated in Fig. 15 to apply a release force in the direction of the arrows A which in turn pulls the local segment 55 in the direction of the arrow B from a rest position retaining the sleeve 6 (Fig. 16) to a release position (Figs. 17 and 18) in which the sleeve 6 is readily pulled and manipulated locally. This arrangement is particularly advantageous in facility local manipulation of the retraction force.

In this embodiment of the invention the outer annular PTFE section 51 is constructed in such a manner that it does not make continuous contact with the inner ring 10 but consists of several local releasable clamps 55 that press the elastomeric material of the sleeve 6 against the inner to anchor the sleeve 6. By releasing the tension between the inner ring 10 and the outer section 51 the elastomeric sleeve 6 in readily slid from one position to another.

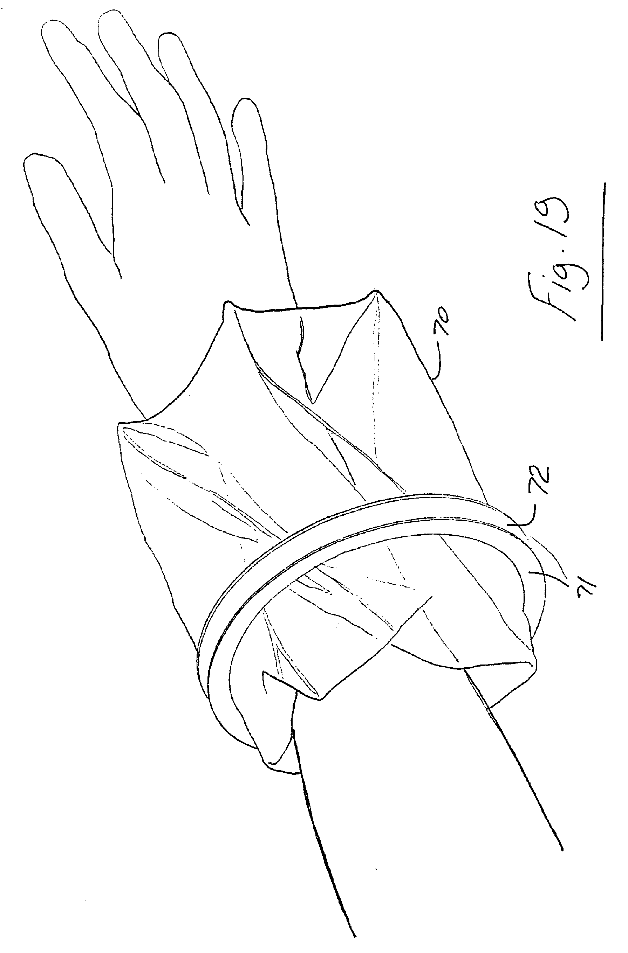

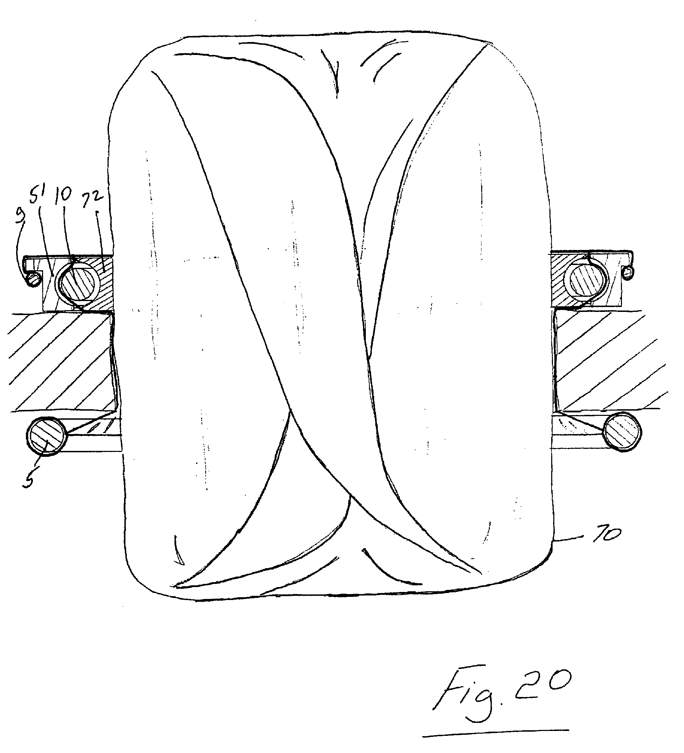

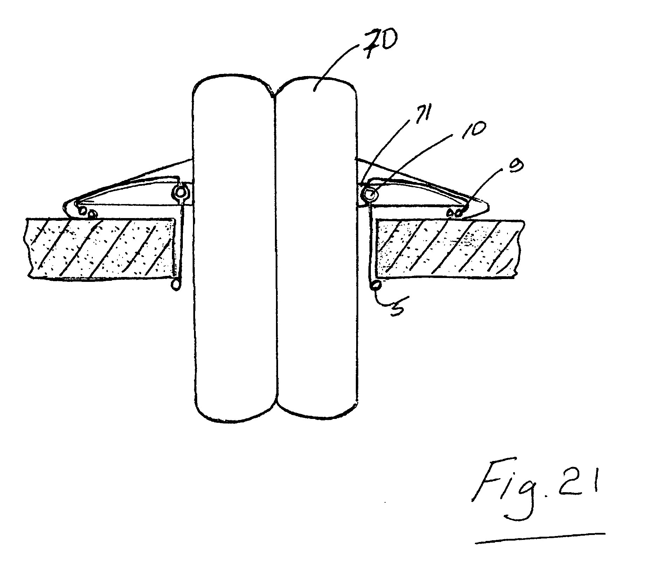

The retractor of the invention also provides a platform on which a wide range of devices may be mounted. In this case the platform is provided by a inner projection part 70 of the guide ring 10 on which a ring part 71 with a complementary recess 72 may be easily fitted somewhat in the manner of a snap-type engagement. Various devices may be provided with such a ring part 71. For example, as illustrated in Figs. 19 to 21 the device may be a hand access device 75 for use in laproscopic surgery. Alternatively the device may be a drape 70 or a form retaining device 85 which is manipulated to the form of an organ to be held back and from which air is the evacuated along an evacuation line 86 to retain the desired organ holding configuration.

In another embodiment of the invention the device the outer PTFE section may be constructed so that it becomes a reverse arrangement of the embodiments described above. In this case there is an outer a ring or other annular shape within which slides the second component. The second component is of the same annular shape as the outer ring but with a lesser diameter and an exterior compartmental recess designed so that the ring fits loosely around the second and both can easily slide relative to each other. The components fit loosely together so that the elastomeric sleeve can fit in the gap between and slide therein.

In general, the retractor is employed by inserting the distal ring 5 of the elastomeric sleeve 6 into an incision and pulling the elastomeric sleeve 6 so that the ring 5 is flat against the interior anatomical surface. The ring 5 prevents the elastomeric sleeve 6 from slipping out of the incision or orifice. The outer PTFE components 10, 11, 51 are then slid down the elastomeric sleeve 6 until they come into contact with the exterior anatomical surface. Retraction is achieved by pulling the elastomeric sleeve 6 taut until the shortening of the distance between the internal ring 5 and the outer ring compartments 10, 11, 51 displaces the elastomeric sleeve 6 laterally and with it the margins of the wound or orifice. The elastomeric sleeve 6 is locked into position by everting it back over the anchor projects 20 components of the device and holding it in place by tension.

The retractor may be used as a platform on which to mount other devices for use in various surgical procedures. Such devices may include: a capping device to cover the incision site of orifice; a hand-access device to allow the surgical procedure to be converted from an open procedure into a hand-assisted laparoscopic procedure; an instrument port for the insertion of instruments; a trocar for use in laparoscopic surgery; an internal organ retractor to assist in the displacement of internal structures from the operative field; or an illuminating means to deliver illumination to an area shaded from the theatre lights. It will be clear to those skilled in the art that the enhancements are not limited to the brief list mentioned here.

The device may be constructed in other annular shapes. For example the distal and proximal annuli on the elastomeric sleeve may be oval or elliptical instead of circular.

The retractor may be used to retract the margins of a natural bodily orifice such as the anus or vagina, or can be used to protect and retract the edges of a man-made stoma such as is created for tracheostomy or following gastrointestinal surgery.

The invention is not limited to the embodiments hereinbefore described which may be varied in both construction and detail.

## Claims

1. A retractor comprising:-
an inner mounting means for mounting through a wound or body opening;
connecting means having a distal end and a proximal end;
the connecting means being mounted to the inner mounting means;
external guide means for the connecting means;
the connecting means being movable relative to the guide means to retract the opening; and
anchor means for anchoring the connecting means to maintain retraction of the opening.

2. A retractor as claimed in claim 1 wherein the connecting member is a sleeve.

3. A retractor as claimed in claim 1 or 2 wherein the guide means comprises an annular ring means.

4. A retractor as claimed in claim 3 wherein the annular ring means comprises inner and outer ring parts between which the connecting means is led.

5. A retractor as claimed in claim 4 wherein the outer ring part includes the anchor means for anchoring the connecting means.

6. A retractor as claimed in claim 5 wherein the anchor means comprises anchor formations on the outer ring to which the connecting means is attached on retraction of the opening.

7. A retractor as claimed in any of claims 4 to 6 wherein the inner ring means defines a projection for engagement in a complementary recess of the outer ring with the connecting means located therebetween.

8. A retractor as claimed in any of claims 4 to 7 wherein at least portion of one of the ring parts is movable from a rest position in which the connecting member is substantially clamped between the ring parts to a release position in which the connecting member is movable relative to the ring parts.

9. A retractor as claimed in claim 8 wherein the outer ring part comprises a plurality of segments which are independently movable to facilitate localised release of the connecting member for adjusting of the retraction force applied at the opening.

10. A retractor as claimed in claim 8 or 9 wherein the ring part or segment thereof are manually manipulable between the clamped rest and release positions.

11. A retractor as claimed in any preceding claim including a platform for attachment of another device to the retractor.

12. A retractor as claimed in any of claims 4 to 10 wherein one of the ring parts defines a platform for attachment of another device to the retractor.

13. A retractor as claimed in any preceding claim wherein the connecting means includes a proximal reinforcing means.

14. A retractor as claimed in claim 13 wherein the proximal reinforcing means is a proximal ring.

15. A retractor as claimed in any preceding claim wherein the distal mounting means is an O-ring.
